# EUROPEAN PATENT SPECIFICATION

(11) **EP 1 796 692 B1**
(45) Date of publication and mention of the grant of the patent: **02.05.2012**
(21) Application number: 05799797.5
(22) Date of filing: 07.09.2005
(51) Int. Cl.: A61K 33/04, A61K 33/30, A61K 33/34, A61K 33/24, A61K 33/32

(54) **TRACE ELEMENTS**
SPURENELEMENTE
OLIGOELEMENTS

(30) Priority: 09.09.2004 ZA 200407201
(43) Date of publication of application: 20.06.2007
(60) Divisional application: 10185852.0
(73) Proprietor: Warburton Technology Limited, Dublin, 2 (IE)
(72) Inventor: LAURIE, Robert Naylor, Private Bag X1 Die Wilgers 0041 (ZA); SMITH, William Alfred Warburton Technology Ltd., Dublin 2 (IE)
(74) Representative: Tetzner, Michael
(86) International application number: PCT/IB2005/052917
(87) International publication number: WO 2006/027745

(56) References cited:
- EP-A1- 1 245 162
- EP-A1- 1 530 908
- WO-A-01/60752
- WO-A-02/17933
- WO-A-2005/105117
- WO-A1-00/47040
- WO-A1-02/069955
- WO-A2-02/34199
- WO-A2-02/47703
- WO-A2-02/067879
- DE-U1- 20 116 346
- DE-U1- 20 202 562
- DE-U1- 20 310 493
- US-A- 4 335 116
- US-A- 4 804 535
- US-A- 5 597 585
- US-A1- 2003 236 203
- US-B1- 6 387 412
- LAURIE R N: "Providing trace elements to livestock - using injectable soln. of EDTA complex of the required mineral" DATABASE WPI, 1983, XP002958133

## Description

The present invention relates to a method of preparing an injectable trace element solution.

In the specification and claims the expression EDTA refers to ethylene diaminotetraacetic acid (C₁₀H₁₆O₈N₂ or (HO₂CH₂C)₂NCH₂CH₂N-(CH₂CO₂H)₂).

US 4,335,116 discloses the preparation of mineral-containing therapeutic compositions containing EDTA complexes of trace elements. In the preparation process (example 1) individual trace element/EDTA solutions are separately prepared in advance and are allowed to stand for some time before they are admixed together to obtain the desired trace element solution.

WO 02/17933 A1 (Example 6) discloses a method of preparing a trace element solution comprising selenium, zinc and manganese and having in total more than 60 mg/ml of metals. This trace element solution is produced in a single continous process by combination of EDTA-complexes with sodium selenite solution. The process starts with the preparation of an EDTA-complex of Zn by adding EDTA, sodium hydroxide and ZnO. In a following step manganese carbonate or manganese hydroxide as powder are added.

Manganese carbonate and manganese hydroxide are susceptible to decomposition and oxidation on storage so that their manganese content varies with time. Using such a stored manganese compound requires additional effort for preparing a trace element solution with a precise manganese content.

It is the object of the invention to provide a simple, time and cost saving method of preparing an injectable trace element solution with a total metal concentration of at least 60 mg/ml and with an accurately defined content of manganese.

According to the invention this object is solved by a method of preparing an injectable trace element solution comprising at least one metal selected from the group comprising selenium, copper, zinc, manganese and chromium, said method including the steps of:
(a) preparing a MnC03 mixture in a container by mixing MnS04 and Na2CO3;
   (b1) adding to the container an EDTA solution selected from the group comprising a potassium EDTA solution and a sodium EDTA solution and
   (b2) subsequently adding at least one metal compound selected from the group comprising ZnO, CuCO3, MnS04 and FeCl3;
(c) adding Na2SeO3 to the container to obtain the trace element solution; and
(d) adding at least one component selected from the group comprising a vitamin, a vaccine, a growth stimulant of the group including zeranol, estradiol, testosterone, progesterone and trenbolone acetate, a dewormer of the group including macrocydic lactones, leramizoles, benzimidazoles and/or salicylanilides, iron dextran and an antibiotic;
(e) wherein the concentration of the metals is at least 60 mg/ml.

A method of the above type (without, however, feature d) is subject of prior International Application PCT/IB2005/051410 (Priority date 3rd May 2004, published 10th November 2005 as WO 2005/105117 A1).

As in the first step a) of the method according to the invention a MnCO₃ mixture is prepared by mixing MnSO₄ and Na₂CO₃ an accurately defined content of manganese is obtained, providing a simple, time and cost saving method which results in an injectable trace element solution having the required total metal concentration of at least 60 mg/ml and containing at least one component added in step d) (vitamin etc.).

The method according to the invention can include the step of adding CrCl₃·6H₂O to the trace element solution. In this case a EDTA/NaOH mixture can be added prior to addition of the CrCl₃·6H₂O to the trace element solution.

The pH of the trace element solution can be adjusted to 6,7 to 7,0. This adjustment can be made by adding at least one compound selected from the group comprising NaOH and EDTA. The addition of this compound can occur gradually with small quantities.

The trace element solution can be diluted.

The temperature of the MnCO₃ mixture is at least 60 degrees Celsius. For this purpose water having a temperature of at least 70 degrees Celsius can be added to the MnCO₃ mixture.

Prior to addition of the Na₂SeO₃ the trace element solution can be cooled.

The vaccine includes antigens or a combination of antigens and adjuvents. The macrocydic lactones (dewormer group) includes doramectin, ivermectin, abamectin and moxidectin.

The invention will now be described by way of examples of the method in accordance with the invention.

### EXAMPLE 1

Example 1 relates to a method to prepare a trace element solution predominantly to be used for cattle and including the mineral elements Selenium, Copper and Chromium.

The method enables preparation of 25 litres of the solution containing 40 mg Zn, 10 mg Mn, 5 mg Se, 15 mg Cu and 5 mg Cr per ml.

### A. Preparing MnCO₃

In a suitable container/drum, the MnCO₃ mud is prepared by adding solutions of 900 g MnSO₄ and 1150 g Na₂CO₃ together. The resultant mixture is decanted and washed three times.

### B. Continuous Batch Process

To the MnCO₃ mud, hot water (70°C) is added to a volume of at least 15 litres. Critical is the temperature at the start of the batch process which should be at least 60°C.

### B.1 Preparing MnEDTA

2000 g EDTA and 500 g NaOH are weighed; the EDTA and NaOH are mixed; the EDTA/NaOH mixture is added to the drum, in small quantities to prevent excessive frothing, until the reaction is complete (leaving a clear pinkish solution).

### B.2 Preparing ZnEDTA (2 steps)

### Step 1:

2600 g EDTA, 690 g NaOH and 700 g ZnO are weighed, the EDTA and NaOH are mixed and ZnO is kept separate. The EDTA/NaOH mixture is added to the drum in small quantities to prevent boiling over, followed by addition of the ZnO. The reaction is allowed to complete (again leaving a clear pink solution). The temperature at this stage could reach 103°C.

### Step 2:

2600 g EDTA, 690 g NaOH and 700 g ZnO are weighed. The EDTA and NaOH are mixed and the ZnO kept separate. The EDTA/NaOH mixture is added to the drum in small quantities to prevent boiling over, where after the ZnO is added. The reaction is allowed to complete (again leaving a clear pink solution). The temperature at this stage could reach 103°C.

### B.3 Preparing CuEDTA

1760 g EDTA, 462 g NaOH and 693 g basic CuCO₃ are weighed. The EDTA and NaOH are mixed and the CuCO₃ kept separate. The EDTA/NaOH mixture is added to the drum, followed by careful addition of the CuCO₃, to prevent excessive frothing, and the reaction is allowed to complete (leaving a clear blue solution).

### B.4 25 g chlorocresol is added and stirred till dissolved.

### B.5 23 litres is made up

### B.6 The mixture is allowed to cool to room temperature.

### C. Final phase

C.1 303 g Na₂SeO₃ is added.
C.2 The pH is adjusted to 6,7 by adding NaOH (40% solution) or EDTA.
C.3 738 g EDTA, 192 g NaOH and 641 g CrCl₃.6H₂O are weighed. The EDTA and NaOH are mixed and added to the drum. The CrCl₃.6H₂O is added, whereby the reaction is slow.
C.4 The volume is made up to 25 litres.

At least one component selected from the group comprising a vitamin, a vaccine, a growth stimulant of the group including zeranol, estradiol, testosterone, progesterone and/or trenbolone acetate, a dewormer of the group including macrocydic lactones, leramizoles, benzimidazoles and/or salicylanilides, iron dextran and an antibiotic is added to the solution and/or blended as an aqueous base; this addition or blending can be done at any stage.

### EXAMPLE 2

Example 2 relates to a method to prepare a trace element solution predominantly to be used for sheep and includes the mineral elements Selenium and Copper.

The method enables preparation of 100 litres of the solution containing 40 mg Zn, 10 mg Mn, 3 mg Se and 10 mg Cu per ml.

### A. Preparing MnCO₃

In a suitable container/drum, the MnCO₃ mud is prepared by adding solutions of 3600 g MnSO₄ and 4600 g Na₂CO₃ together. The mixture is decanted and washed three times.

### B. Continuous Batch Process

To the MnCO₃ mud, is added hot water (70°C) to a volume of at least 60 litres. The temperature at the start of the batch process is critical and should be at least 60°C.

### B.1 Preparing MnEDTA

8000 g EDTA and 2000 g NaOH are weighed. The EDTA and NaOH are mixed. The EDTA/NaOH mixture is added to the drum, in small quantities to prevent excessive frothing, until the reaction is complete (leaving a clear pinkish solution).

### B.2 Preparing ZnEDTA (2 steps)

### Step 1:

10400 g EDTA, 2760 g NaOH and 2800 g ZnO are weighed. The EDTA and NaOH are mixed and the ZnO kept separate. The EDTA/NaOH mixture is added to the drum in small quantities to prevent boiling over, followed by addition of the ZnO. The reaction is allowed to complete (again leaving a clear pink solution). The temperature at this stage could reach 103°C.

### Step 2:

10400 g EDTA, 2760 g NaOH and 2800 g ZnO are weighed. The EDTA and NaOH are mixed and the ZnO kept separate. The EDTA/NaOH mixture is added to the drum in small quantities to prevent boiling over, followed by addition of the ZnO. The reaction is allowed to complete (again leaving a clear pink solution). The temperature at this stage could reach 103°C.

### B.3 Preparing CuEDTA

4646 g EDTA, 1220 g NaOH and 1835 g basic CuCO₃ are weighed. The EDTA and NaOH are mixed and the CuCO₃ kept separate. The EDTA/NaOH mixture is added to the drum, followed by careful addition of the CuCO₃, to prevent excessive frothing, and the reaction is allowed to complete (leaving a clear blue solution).

### B.4 100 g chlorocresol is added and the mixture stirred until dissolved.

### B.5 The volume is made up to 96 litres

### B.6 The mixture is cooled to room temperature.

### C. Final phase

C.1 728 g Na₂SeO₃ is added.
C.2 The pH is adjusted to 6,7 by adding NaOH (40% solution) or EDTA.
C.3 The volume is made up to 100 litres.

At least one component selected from the group comprising a vitamin, a vaccine, a growth stimulant of the group including zeranol, estradiol, testosterone, progesterone and/or trenbolone acetate, a dewormer of the group including macrocydic lactones, leramizoles, benzimidazoles and/or salicylanilides, iron dextran and an antibiotic is added to the solution and/or blended as an aqueous base; this addition or blending can be done at any stage.

### EXAMPLE 3

Example 3 relates to a method to prepare a trace element solution predominantly to be used for cattle and includes the mineral elements Selenium and Copper.

The method enables preparation of 100 litres of the solution containing 40 mg Zn, 10 mg Mn, 5 mg Se and 15 mg Cu per ml.

### A. Preparing MnCO₃

In a suitable container/drum, the MnCO₃ mud is prepared by adding solutions of 3600 g MnSO₄ and 4600 g Na₂CO₃ together. The mixture is decanted and washed three times.

### B. Continuous Batch Process

To the MnCO₃ mud, hot water (70°C) is added to a volume of at least 60 litres. The temperature at the start of the batch process is critical and should be at least 60°C.

### B.1 Preparing MnEDTA

7840 g EDTA and 1960 g NaOH are weighed. The EDTA and NaOH are weighed. The EDTA/NaOH mixture is added to the drum, in small quantities to prevent excessive frothing, until the reaction is complete (leaving a clear pinkish solution).

### B.2 Preparing ZnEDTA (2 steps)

### Step 1:

10400 g EDTA, 2760 g NaOH and 2800 g ZnO are weighed. The EDTA and NaOH are mixed and the ZnO kept separate. The EDTA/NaOH mixture is added to the drum, in small quantities to prevent boiling over, followed by addition of the ZnO. The reaction is allowed to complete (again leaving a clear pink solution). The temperature at this stage could reach 103°C.

### Step 2:

10400 g EDTA, 2760 g NaOH and 2800 g ZnO are weighed. The EDTA and NaOH are mixed and the ZnO kept separate. The EDTA/NaOH mixture is added to the drum, in small quantities to prevent boiling over, followed by addition of the ZnO. The reaction is allowed to complete (again leaving a clear pink solution). The temperature at this stage could reach 103°C.

### B.3 Preparing CuEDTA

7040 g EDTA, 1848 g NaOH and 2780 g basic CuCO₃ are weighed. The EDTA and NaOH are mixed and the CuCO₃ kept separate. The EDTA/NaOH mixture is added to the drum, followed by careful addition of the CuCO₃, to prevent excessive frothing, and the reaction is allowed to complete (leaving a clear blue solution).

### B.4 100 g chlorocresol is added and the mixture stirred till dissolved.

### B.5 The mixture is made up to 96 litres

### B.6 The mixture is allowed to cool to room temperature.

### C. Final phase

C.1 1212 g Na₂SeO₃ is added.
C.2 The pH is adjusted to 7,0 by adding NaOH (40% solution) or EDTA.
C.3 The volume is made up to 100 litres.

At least one component selected from the group comprising a vitamin, a vaccine, a growth stimulant of the group including zeranol, estradiol, testosterone, progesterone and/or trenbolone acetate, a dewormer of the group including macrocydic lactones, leramizoles, benzimidazoles and/or salicylanilides, iron dextran and an antibiotic is added to the solution and/or blended as an aqueous base; this addition or blending can be done at any stage.

## Claims

1. A method of preparing an injectable trace element solution comprising at least one metal selected from the group comprising selenium, copper, zinc, manganese and chromium, said method including the steps of:
(a) preparing a MnCO₃ mixture in a container by mixing MnSO₄ and Na₂CO₃;
(b1) adding to the container an EDTA solution selected from the group comprising a potassium EDTA solution and a sodium EDTA solution and
(b2) subsequently adding at least one metal compound selected from the group comprising ZnO, CuCO₃, MnSO₄ and FeCl₃;
(c) adding Na₂SeO₃ to the container to obtain the trace element solution; and
(d) adding at least one component selected from the group comprising a vitamin, a vaccine, a growth stimulant of the group including zeranol, estradiol, testosterone, progesterone and trenbolone acetate, a dewormer of the group including macrocydic lactones, leramizoles, benzimidazoles and/or salicylanilides, iron dextran and an antibiotic;
(e) wherein the concentration of the metals is at least 60 mg/ml.

2. A method as claimed in claim 1, which includes the step of adding CrCl₃.6H₂O to the trace element solution.

3. A method as claimed in claim 2, which includes the step of adding a EDTA/NaOH mixture prior to addition of the CrCl₃.6H₂O to the trace element solution.

4. A method as claimed in claim 1, which includes the step of adjusting the pH of the trace element solution to 6,7 to 7,0.

5. A method as claimed in claim 4, which includes the step of adjusting the pH of the trace element solution by adding at least one compound selected from the group comprising NaOH and EDTA.

6. A method as claimed in claim 1, which includes the step of diluting the trace element solution.

7. A method as claimed in claim 1, in which the temperature of the MnCO₃ mixture is at least 60 degrees Celsius.

8. A method as claimed in claim 7, which includes the step of adding water having a temperature of at least 70 degrees Celsius to the MnCO₃ mixture.

9. A method as claimed in claim 5, in which the addition of the compound selected from the group comprising NaOH and EDTA occurs gradually with small quantities.

10. A method as claimed in claim 1, which includes the step of cooling the trace element solution prior to addition of the Na₂SeO₃.

## Patentansprüche

1. Verfahren zur Herstellung einer injizierbaren Spurenelementlösung, enthaltend wenigstens ein Metall ausgewählt aus der Gruppe, die Selen, Kupfer, Zink, Mangan und Chrom enthält, wobei das Verfahren die folgenden Schritte umfasst:
(a) Herstellen einer MnCO₃-Mischung in einem Behälter durch Mischen von MnSO₄ und Na₂CO₃,
(b1) Hinzufügen einer EDTA-Lösung zu dem Behälter, die aus der Gruppe ausgewählt ist, die eine Kalium-EDTA-Lösung und eine Natrium-EDTA-Lösung enthält, und
(b2) anschließend Hinzufügen wenigstens einer Metallverbindung, ausgewählt aus der Gruppe, die ZnO, CuCO₃, MnSO₄, und FeCl₃ enthält,
(c) Hinzufügen von Na₂SeO₃ zum Behälter, um die Spurenelementlösung zu erhalten, und
(d) Hinzufügen wenigstens einer Komponente, die aus der Gruppe ausgewählt ist, die ein Vitamin, einen Impfstoff, ein Wachstumsstimulans aus der Gruppe, die Zeranol, Estradiol, Testosteron, Progesteron und Trenbolonacetat enthält, ein Entwurmungsmittel aus der Gruppe, die makrozyklische Laktone, Leramizol, Benzimidazole und/oder Salizylanilide enthält, Eisendextran und ein Antibiotikum enthält,
(e) wobei die Konzentration der Metalle wenigstens 60 mg/ml beträgt.

2. Verfahren nach Anspruch 1, das den Schritt des Hinzufügens von CrCl₃·6H₂O zu der Spurenelementlösung enthält.

3. Verfahren nach Anspruch 2, das den Schritt des Hinzufügens einer EDTA/NaOH-Mischung vor der Zugabe von CrCl₃·6H₂O zu der Spurenelementlösung enthält.

4. Verfahren nach Anspruch 1, das den Schritt des Einstellens des pH der Spurenelementlösung auf 6,7 bis 7,0 enthält.

5. Verfahren nach Anspruch 4, das den Schritt des Einstellens des pH der Spurenelementlösung durch Hinzufügen wenigstens einer Komponente enthält, die aus der Gruppe ausgewählt ist, die NaOH und EDTA enthält.

6. Verfahren nach Anspruch 1, das den Schritt des Verdünnens der Spurenelementlösung enthält.

7. Verfahren nach Anspruch 1, bei dem die Temperatur der MnCO₃-Mischung wenigstens 60 Grad Celsius beträgt.

8. Verfahren nach Anspruch 7, das den Schritt des Hinzufügens von Wasser, das eine Temperatur von wenigstens 70 Grad Celsius aufweist, zu der MnCO₃-Mischung enthält.

9. Verfahren nach Anspruch 5, bei dem das Hinzufügen der Verbindung, die aus der Gruppe ausgewählt ist, die NaOH und EDTA enthält, nach und nach mit kleinen Mengen geschieht.

10. Verfahren nach Anspruch 1, das den Schritt des Kühlens der Spurenelementlösung vor dem Hinzufügen des Na₂SeO3 enthält.

## Revendications

1. Procédé de préparation d'une solution d'oligoéléments injectable, comprenant au moins un métal choisi dans le groupe formé de : sélénium, cuivre, zinc, manganèse et chrome, ledit procédé comprenant les étapes consistant à:
(a) préparer un mélange de MnCO₃ dans un contenant, par mélange de MnS04 et de Na₂CO₃ ;
(b1) ajouter au contenant une solution d'EDTA choisie dans le groupe formé d'une solution d'EDTA potassique et une solution d'EDTA sodique et
(b2) ajouter ensuite au moins un composé métallique choisi dans le groupe formé de ZnO, CuCO₃, MnSO₄ et FeCl₃ ;
(c) ajouter Na₂SeO₃ au contenant, pour obtenir la solution d'oligoéléments ; et
(d) ajouter au moins un composant choisi dans le groupe formé de : une vitamine, un vaccin, un stimulateur de croissance du groupe comprenant acétate de trenbolone, zéranol, estradiol, testostérone et progestérone, un vermifuge du groupe comprenant lactones macrocycliques, léramizoles, benzimidazoles et/ou salicylanilides, dextrane de fer et un antibiotique;
(e) la concentration des métaux étant d'au moins 60 mg/ml.

2. Procédé selon la revendication 1, qui comprend l'étape consistant à ajouter CrCl₃.6H₂O à la solution d'oligoéléments.

3. Procédé selon la revendication 2, qui comprend l'étape consistant à ajouter un mélange EDTA/NaOH avant l'addition du CrCl₃.6H₂O à la solution d'oligoéléments.

4. Procédé selon la revendication 1, qui comprend l'étape consistant à ajuster le pH de la solution d'oligoéléments à 6,7 à 7,0.

5. Procédé selon la revendication 4, qui comprend l'étape consistant à ajuster le pH de la solution d'oligoéléments par addition d'au moins un composé choisi dans le groupe formé de NaOH et EDTA.

6. Procédé selon la revendication 1, qui comprend l'étape consistant à diluer la solution d'oligoéléments.

7. Procédé selon la revendication 1, dans laquelle la température du mélange de MnCO₃ est d'au moins 60 degrés Celsius.

8. Procédé selon la revendication 7, qui comprend l'étape consistant à ajouter de l'eau à une température d'au moins 70 degrés Celsius au mélange de MnCO₃.

9. Procédé selon la revendication 5, dans lequel l'addition du composé choisi dans le groupe formé de NaOH et EDTA se fait progressivement, par petites quantités.

10. Procédé selon la revendication 1, qui comprend l'étape consistant à refroidir la solution d'oligoéléments avant addition au Na₂SeO₃.
